# EUROPÄISCHE PATENTANMELDUNG

(11) **EP 4 331 558 A1**
(43) Veröffentlichungstag der Anmeldung: **06.03.2024**
(21) Anmeldenummer: 22193140.5
(22) Anmeldetag: 31.08.2022
(51) Int. Cl.: A61K 6/30

(54) **BIOKOMPATIBLE SCHMELZ-DENTIN-EINKOMPONENTENADHÄSIVE**

(71) Anmelder: Ivoclar Vivadent AG, 9494 Schaan (LI)
(72) Erfinder: Köhler, Thomas, 78479 Reichenau (DE); Barbisch, Michael, 6833 Klaus (AT); Bock, Thorsten, 6800 Feldkirch (AT); HARTWIG, Andreas, 27721 Ritterhude (DE); RISCHKA, Klaus, 21225 Tostedt (DE)
(74) Vertreter: Uexküll & Stolberg

(57) **Zusammenfassung**

Die Erfindung betrifft einen Dentalwerkstoff, der mindestens ein Copolymer enthält, das durch (I) Copolymerisation eines oder mehrerer Monomere M_{G} mit einer polymerisationsfähigen (Meth)acrylatgruppe, eines oder mehrerer OH-Gruppen-haltiger (Meth)acrylate M_{F}, eines oder mehrerer stark saurer Haftmonomere M_{H} und ggf. eines oder mehrerer polymerisationsfähiger Carbonsäure-Monomere M_{CS}, (II) Umsetzen des erhaltenen Copolymers A₀ in einer polymeranalogen Reaktion mit einem funktionalisierten, radikalisch polymerisationsfähigen Monomer M_{P}, das mit den OH-Gruppen des Monomers M_{F} in der Polymerkette von A₀ reagiert und dabei ein Copolymer A_{P} mit seitenständigen polymerisationsfähigen Gruppen bildet, und (III) Neutralisieren des Copolymers A_{P} mit einer Base erhältlich ist. Der Werkstoff eignet sich besonders als dentales Adhäsiv.

## Beschreibung

Die vorliegende Erfindung betrifft Dentalwerkstoffe auf der Basis von Copolymeren mit adhäsiven Eigenschaften, die sich insbesondere als Adhäsive für direkte oder indirekte Dentalrestaurationen eignen.

In der Zahnmedizin werden Schmelz-Dentin-Adhäsive vor allem zur Herstellung eines festen und dauerhaften Verbundes zwischen direkten oder indirekten Füllungsmaterialien und der Zahnhartsubstanz (Schmelz und Dentin) eingesetzt. Sie enthalten in der Regel eine Mischung verschiedener radikalisch polymerisierbarer Methacrylatmonomere, Polymere, ein Initiatorsystem, Lösungsmittel, wie wässriges Ethanol oder Aceton, und weitere Zusätze, wie z.B. Stabilisatoren und Rheologie-Additive. Die Monomermischung enthält ihrerseits meist ein stark saures Haftmonomer, beispielsweise ein methacrylgruppenhaltiges Dihydrogenphosphat mit selbstätzenden Eigenschaften, das eine starke Anbindung an die Zahnhartsubstanz gewährleistet, Dimethacrylate, wie BisGMA und Triethylenglycoldimethacrylat (TEGDMA), die eine schnelle Ausbildung einer stabilen Adhäsivschicht begünstigen, und hydrophile Monomere, wie z.B. 2-Hydroxyethylmethacrylat (HEMA), zur Förderung des Penetrationsvermögens. Polymere, wie. z.B. methacrylierte Polyacrylsäure oder Itaconsäurecopolymere, sollen die Filmbildung der Adhäsive verbessern und die Technik-Sensitivität verringern. Als Polymerisationsinitiatoren für die Härtung mit sichtbarem Licht werden vor allem Mischungen von Campherchinon (CQ) mit einem tertiären Amin, wie z.B. 4-Dimethylaminobenzoesäureethylester (EMBO), verwendet, die in dualhärtenden Systemen mit einem Redoxinitiatorsystem kombiniert werden.

Die US 5,700,875 offenbart dentale Adhäsive, die 60 bis 98 Gew.-% einer polymerisierbaren Monomermischung, 2 bis 40 Gew.-% Polymer und 0,01 bis 35 Gew.-% eines Polymerisationsinitiators enthalten. Die Monomermischung enthält 2 bis 25 Gew.-% saure Monomeren mit Sulfonsäure-, Carbornsäure-, Carbonsäureanhydrid- oder Phosphorsäuregruppen. Das Polymer ist ein Copolymer auf der Basis von Alkyl(meth)acrylaten, Styrolmonomeren, Hydroxyalkyl(meth)acrylaten, Butadien, Polyalkylmethacrylaten und/oder Polyvinylacetat und ist in der Monomermischung gelöst oder dispergiert. Die Adhäsive sollen gut handhabbar sein und eine ausgezeichnete Haftung aufweisen.

R.R. Moräes, J.W. Garcia, N.D. Wilson, S.H. Lewis, M.D. Barros, B. Yang, C.S. Pfeifer, J.W. Stansbury, J. Dent. Res. 91 (2012):179-184, untersuchen die Wirkung der Zugabe von Nanogelpartikeln auf Dentaladhäsive. Hierzu wurden methacrylatfunktionalisierte Nanogele aus Isobornylmethacrylat und UDMA bzw. ethoxyliertem BisGMA mit einer Partikelgröße von 10 bis 100 nm in Konzentrationen von 17 Gew.-% und 23 Gew.-% in einer BisGMA-HEMA-Ethanol-Mischung dispergiert und anschließend die mechanischen Eigenschaften sowie die Dentinhaftung bestimmt. Es wurden verbesserte mechanische Eigenschaften und Dentinhaftwerte gemessen.

Die US 2013/0224692 A1 offenbart dentale Adhäsive, die bis zu 15 Gew.-% eines lichthärtenden Ionomers, röntgenopake Metalloxid-Nanopartikel, ein phosphorhaltiges saures Monomer, weitere Monomere, Wasser, ein polares Lösungsmittel und Initiator enthalten. Unter lonomeren werden Polymere mit ionischen und radikalisch polymerisierbaren Gruppen verstanden, wie z.B. Polyalkencarbonsäuren, die durch Homo- oder Copolymerisation von ungesättigten Mono-, Di- oder Tricarbonsäuren erhalten und mit isocyanatgruppenhaltigen Monomeren modifiziert werden.

Die EP 3 120 827 A1 offenbart radikalisch polymerisierbare Dentalwerkstoffe, die sich als dentale Adhäsive, Beschichtungsmaterialien, Füllungskomposite und Zemente eignen. Die Werkstoffe enthalten mindestens ein radikalisch polymerisierbares (Meth)acrylatcopolymer mit stark sauren Phosphonsäureguppen und einer zahlenmittlere Molmasse von 1.000 bis 200.000 g/mol, radikalisch polymerisierbare Monomere mit und ohne Säuregruppe sowie einen Initiator für die radikalische Polymerisation. Durch die Kombination von aciden Polymeren und aciden Monomeren wird die Haftung an Zahnschmelz und Dentin optimiert.

Nachteilig an bekannten Dentaladhäsiven ist, dass sie nach der Härtung in der Regel einen mehr oder weniger großen Restmonomergehalt aufweisen. Obwohl die eingesetzten Monomeren nicht systemisch toxisch sind, können sie doch unerwünschte Effekte zeigen und zu kontaktallergischen Reaktionen führen.

Zur Verbesserung der Biokompatibilität werden im Stand der Technik verschiedene Konzepte vorgeschlagen, vor allem Bioadhäsive, die von Muschelklebern inspiriert sind, und sog. Ormocer-haltige Adhäsive. Ormocere (organically modified ceramics) sind Cokondensate (Heteropolysiloxane) von Di- oder Trialkoxysilanen, die organischen Gruppen enthalten, und ggf. Ti- oder Zr-Alkoxiden.

Die DE 196 43 007 A1 offenbart Haftvermittler und Klebstoffe auf der Basis von Umsetzungsprodukten von Peptiden oder Proteinen aus den Byssusfäden der Miesmuschel mit radikalisch polymerisierbaren Monomeren, die für dentale Zwecke geeignet sein sollten.

Die WO 2006/045034 A1 betrifft dentale Primer, die 0,1 bis 50 Gew.-% DOPA (3,4-Dihydroxyphenyl-L-alanin) in Kombination mit verdünnter Mineralsäure enthalten. Das DOPA soll das gleichzeitige Grundieren und Ätzen des Zahns vor dem Aufbringen eines Restaurationsmaterials ermöglichen.

Die US 2011/0288252 A1 offenbart Heteropolysiloxane (Ormocere), auf der Basis von Dialkoxy- und Dihydroxyphenyl-Gruppen haltigen Silanen, die mit DOPA-Gruppen modifiziert sind, zur Herstellung von Adhäsiven. Die Heteropolysiloxane können durch hydrolytische Kondensation mit Wasser oder durch radikalische Polymerisation ggf. vorhandener polymerisationsfähiger Gruppen härten.

Nachteilig an solchen Bioadhäsiven ist ihre relativ geringe Haftwirkung und der lange Zeitraum, oft Stunden der Tage, der erforderlich ist, um die volle Haftwirkung zu erreichen. Sie haben daher als Schmelz-Dentin-Adhäsive keine praktische Anwendung gefunden.

Unter Biokompatibilität versteht man im Allgemeinen den Grad der Gewebeverträglichkeit eines im Körper eingesetzten oder mit seiner Oberfläche in Kontakt kommenden Werkstoffes. Biokompatible Materialien sollen sich gegenüber dem Körper neutral verhalten und zu keiner negative Beeinflussung führen. Insbesondere dürfen von ihnen keine toxikologischen, allergischen, mutagenen, teratogenen oder kanzerogenen Effekte ausgehen. Dentalwerkstoffe dürfen orale und andere Körpergewebe nicht reizen.

Die DE 10 2018 204 655 A1 offenbart Glasionomerzemente, die ein säurereaktives Pulver, eine mehrprotonige Säure, Wasser und dispergierte Polymerpartikel enthalten. Bevorzugte Polymerpartikel sind Polyurethanpartikel. Diese haben vorzugsweise eine mittlere Partikelgröße von weniger als 1 µm. Die Partikel werden vorzugsweise als Primär- oder Sekundärdispersion hergestellt. Die Polymerpartikel sollen toxikologisch unbedenklich sein und die Riss- und Bruchzähigkeit der Zemente verbessern. Die Materialien enthalten vorzugsweise keine polymerisierbaren Verbindungen, wie z.B. (Meth)acrylate.

Unter einer Primärdispersion versteht ein Fachmann eine Polymerdispersion, die dadurch erhalten wird, dass Monomere zunächst in Wasser emulgiert und dann in der dispergierten Phase polymerisiert werden. Zur Herstellung von Primärdispersionen sind Emulgatoren oder Schutzkolloide erforderlich. Zur Herstellung einer Sekundärdispersion wird ein Polymer durch Lösungspolymerisation in einem flüchtigen organischen Lösungsmittel herstellt. Die erhaltene Polymerlösung wird anschließend in einer wässrigen Phase dispergiert und dann das Lösungsmittel entfernt. Saure oder basische Gruppen des Polymers werden vor dem Dispergieren neutralisiert. Die bei der Neutralisation entstehenden Salzgruppen stabilisieren die Polymerpartikel in der Dispersion. Je nach Ladung der Polymerpartikel unterscheidet man anionisch oder kationisch stabilisierte Sekundärdispersionen. Zur Herstellung von anionischen Sekundärdispersionen werden beispielsweise Copolymere mit sauren Gruppen, z.B. Carboxylgruppen, eingesetzt, die durch Zugabe von Basen neutralisiert werden. Die Stabilisierung der Sekundärpolymerdispersion wird in diesem Fall durch Carboxylatgruppen an der Oberfläche der Polymerpartikel bewirkt. Werden Copolymere mit basischen Gruppen, wie z.B. tertiären Aminogruppen eingesetzt, erhält man kationische Sekundärdispersionen, wobei die Neutralisation hier durch die Zugabe einer Säure erfolgt.

Sekundärdispersionen zeichnen sich dadurch aus, dass sie weder Emulgatoren noch Schutzkolloide benötigen. Emulgatoren erhöhen die Wasserdampfdurchlässigkeit und Wassersorption von Polymerbeschichtungen. Y. Liu, W.-J. Soer, J. Scheerder, G. Satgurunathan, J.L. Keddie, ACS Applied Materials & Interfaces 7 (2015) 12147-12157, haben gezeigt, dass emulgatorfreie Sekundärdispersionen aus 2-Ethylhexylacrylat, Acrylsäure und n-Butylmethacrylat eine geringere Wassersorption und einen niedrigeren Wasserdiffusionskoeffizienten und damit bessere Barriereeigenschaften als analoge Beschichtungen mit einem vergleichbaren Emulsionspolymerisat aufweisen.

Die EP 0 305 850 A2 offenbart wässrige Sekundärdispersionen auf der Basis von Phosphatgruppen enthaltenden Copolymeren, die durch Polymerisation einer Mischung von 25 bis 98 Gew.-% (Meth)acrylsäureestern, 1 bis 10 Gew.-% (Meth)acrylsäure, 1 bis 8 Gew.-% eines Phosphorsäureesterphosphats mit einer radikalisch polymerisierbaren Gruppe und ggf. weiteren olefinisch ungesättigten Verbindungen erhalten werden. Die Dispersionen sollen sich zur Herstellung von mit Aktivpigmenten gefüllten Anstrichstoffen zum Korrosionsschutz eignen.

Die EP 0 841 352 A1 betrifft ein Verfahren zur Herstellung von Poly(meth)acrylatsekundärdispersionen, die sich durch eine hohe Lagerstabilität auszeichnen sollen. Die Dispersionen werden durch Copolymerisation von einfach ethylenisch ungesättigten Monomeren, wie Acrylsäure- oder Methacrylsäurealkylestern, ethylenisch ungesättigten Monomeren, die alkoholische Hydroxylgruppen tragen, carboxylgruppenhaltigen, ungesättigten Monomeren, wie Acryl- oder Methacrylsäure, und ggf. einem Regler in einem organischen Lösungsmittel erhalten. Nach Abschluss der Copolymerisation soll der Anteil wasserlöslicher Restmonomere im Polymerisationsansatz unter 0,1 Gew.-% liegen. Die Dispersionen sollen sich als Bindemittel in wasserverdünnbaren Beschichtungsmitteln eignen, beispielsweise für Grundierungen, Decklacke oder Klarlacke für Holz-, Metall- oder Kunststoffbeschichtungen.

Die EP 1 270 619 A2 offenbart wässrige Vinylpolymer-Sekundärdispersionen, die mindestens ein amphiphiles Polymer enthalten, das aus einem hydrophoben und einem hydrophilen Segment aufgebaut ist. Das amphiphile Polymer soll den Dispersionen eine ausreichende Thermostabilität verleihen. Die Dispersionen werden durch Polymerisation einer Mischung ethylenisch ungesättigter Monomerer in Gegenwart eines Initiators in einem Wasser-Lösungsmittel-Gemisch unter Zusatz mindestens eines amphiphilen Polymers hergestellt. Die Monomermischung enthält einfach ethylenisch ungesättigte Monomere, wie Acrylsäure- oder Methacrylsäurealkylester, ethylenisch ungesättigten Monomere, die alkoholische Hydroxylgruppen tragen, und carboxylgruppenhaltige, ungesättigten Monomere, wie Acryl- oder Methacrylsäure. Sie kann außerdem Monomere mit Sulfonsäuregruppen enthalten. Die Dispersionen sollen sich als Bindemittel oder Bindemittelkomponente in wasserverdünnbaren Beschichtungsmitteln eignen, beispielsweise für Grundierungen, Füller, Decklacke, Klarlacke, Hochglanzlacke und Einschichtlacke z.B. in der Industrielackierung, Automobilerst- und Reparaturlackierung.

B. Schlarb, M. G. Rau, S. Haremza, Prog. Org. Coat. 26 (1995) 207-215, haben gezeigt, dass sich durch Mischen von zwei Copolymeren mit unterschiedlichem Salzgruppengehalt Sekundärdispersionen herstellen lassen, die die Herstellung hydrophober Beschichtungen ermöglichen. Sie haben ein hydrophiles Copolymer mit Salzgruppen mit einem hydrophoben Copolymer ohne Salzgruppen kombiniert. Das hydrophile Copolymer besteht aus 10 Gew.-% Acrylsäure, 53 Gew.-% Butylacrylat und 37 Gew.-% Styrol, das hydrophobe Copolymer aus 49 Gew.-% n-Butylacrylat, 37 Gew.-% Styrol und 16 Gew.-% Methylmethacrylat. Die Beschichtungen sollen sich durch eine geringe Wasseraufnahme auszeichnen und sich besonders zur Herstellung von Korrosionsschutzfarben und Farben für Fahrbahnmarkierungen eignen.

In einer späteren Publikation, B. Schlarb, S. Haremza, W. Heckmann, B. Morrison, R. Müller-Mall, M. G. Rau MG, Prog. Org..Coat. 29 (1996) 201-208, haben die Autoren gezeigt, dass durch Variation der Anteile und der Zusammensetzung der Copolymere Polymerlatexpartikel mit Kern-Schale-Struktur hergestellt werden können.

Ö. Kutlug, S. Reck, A. Hartwig, Int. J. Adhes. Adhes., 91 (2019) 36-42, beschreiben vernetzbare Haftklebstoffe auf der Basis einer Sekundärdispersion mit zwei unterschiedlichen Copolymeren. Die Dispersion erhält ein Carboxycopolymer aus 10 Gew.-% Acrylsäure und 90 Gew.-% 2-Ethylhexylacrylat und ein Epoxycopolymer aus 15 Gew.-% Glycidylmethacrylat, 20 Gew.-% Hydroxyethylmethacrylat und 65 Gew.-% 2-Ethylhexylmethacrylat. Die Haftkleber sollten eine verbesserte Vernetzung und als Folge davon eine bessere Scherfestigkeit zeigen.

Der Erfindung liegt die Aufgabe zugrunde, biokompatible Dentalwerkstoffe zur Verfügung zu stellen, die sich besonders als Adhäsive für dentale Anwendungen eignen, insbesondere als Adhäsive zur Befestigung direkter und indirekter dentaler Restaurationen an der Zahnhartsubstanz, d.h. an Zahnschmelz und Dentin. Die Dentalwerkstoffe sollen insbesondere unter oralen Bedingungen eine gute Haftung auf der Zahnhartsubstanz gewährleisten.

Die Aufgabe wird erfindungsgemäß durch Dentalwerkstoffe gelöst, die mindestens ein **Copolymer A_{P}** enthalten, das erhältlich ist durch
(I) Copolymerisation von
   (a) einem oder mehreren Monomeren M_{G} mit einer (1) radikalisch polymerisationsfähigen (Meth)acrylatgruppe,
   (b) einem oder mehreren OH-Gruppen-haltigen (Meth)acrylaten M_{F},
   (c) einem oder mehreren stark saure Haftmonomeren M_{H} und
   (d) ggf. einem oder mehreren polymerisationsfähigen Carbonsäure-Monomeren M_{CS}, das oder die eine (1) radikalisch polymerisationsfähige Gruppe enthalten,
(II) Umsetzen des in Schritt (I) erhaltenen Copolymers A₀ in einer polymeranalogen Reaktion mit mindestens einem funktionalisierten, radikalisch polymerisationsfähigen Monomer M_{P}, das mit den OH-Gruppen des Monomers M_{F} in der Polymerkette von A₀ reagiert und dabei ein Copolymer A_{P} bildet, das seitenständige polymerisationsfähige Gruppen trägt, und
(III) Neutralisieren des Copolymers Aₚ mit einer Base.

Zur Herstellung des Copolymers A₀ werden in allen Fällen ausschließlich monofunktionelle Monomere eingesetzt, d.h. Monomere mit einer radikalisch polymerisationsfähigen Gruppe. Polyfunktionelle Monomere, d.h. Monomere mit zwei oder mehr radikalisch polymerisationsfähigen Gruppen, würden bei der Polymerisation eine Vernetzung und damit die Bildung unlöslicher Copolymere bewirken.

Das Copolymer A₀ weist vorzugsweise die folgende Zusammensetzung auf:
(a) 20 bis 80 Gew.-%, besonders bevorzugt 40 bis 70 Gew.-% Monomer M_{G},
(b) 1 bis 70 Gew.-%, besonders bevorzugt 5 bis 40 Gew.-% Monomer M_{F},
(c) 1 bis 40 Gew.-%, besonders bevorzugt 5 bis 30 Gew.-% Monomer M_{H} und
(d) 0 bis 30 Gew.-%, besonders bevorzugt 5 bis 20 Gew.-% Monomer M_{CS},
wobei sich alle Prozentangaben auf die Masse des Copolymers A₀ beziehen. Besonders bevorzugt sind Copolymere, bei denen die Summe von (a), (b), (c) und (d) 100% beträgt.

Die erfindungsgemäßen Dentalwerkstoffe eignen sich besonders als dentale Restaurationsmaterialien, insbesondere als dentale Adhäsive und ganz besonders als Schmelz-Dentin-Adhäsive und werden im Folgenden daher der Einfachheit halber auch als Adhäsive bezeichnet.

Gemäß einer bevorzugten Ausführungsform der Erfindung wird das Copolymer Aₚ in Form einer Sekundärdispersion hergestellt. Hierzu wird die Copolymerisation der Monomere M_{G}, M_{F}, M_{H} und ggf. M_{CS} zu Copolymer A₀ gemäß Schritt (I) in einem organischen Lösungsmittel durchgeführt, nach Umsetzung des Copolymers A₀ mit Monomer Mₚ (Schritt (II)) und anschließender Neutralisation (Schritt (III)) wird
(IV) die in Schritt (III) erhaltenen Copolymer-Lösung ggf. mit Wasser versetzt und gerührt, und
(V) dann das organische Lösungsmittel ggf. abdestilliert.

Die Zugabe von Wasser in Schritt (IV) kann entfallen, wenn zur Neutralisation eine wässrige Lösung der Base verwendet wird, die eine zum Dispergieren der Polymerlösung ausreichende Menge an Wasser enthält. Das Rühren in Schritt (IV) wird so durchgeführt, dass die Copolymer-Lösung aus Schritt (III) in der wässrigen Phase dispergiert wird.

Als **Monomer M_{G}** werden vorzugsweise (Meth)acrylate eingesetzt, die bei der Homopolymerisation Polymere mit einer Glasübergangstemperatur T_{G} von unter 40°C ergeben. In der Polymeranalytik übliche Methoden zur Bestimmung der Glasübergangstemperatur T_{G} sind die dynamische Differenzkalorimetrie DSC (Differential Scanning Calorimetrie) und die dynamisch-mechanische Analyse DMA. Vorzugsweise wird die Glasübergangstemperatur T_{G} mit der DMA gemäß ISO-6721-11 aus 2019 bestimmt.

Besonders bevorzugte Monomere M_{G} sind Alkyl- und Cycloalkyl(meth)acrylate, insbesondere lineare oder verzweigte C₁-C₂₅-Alkyl(meth)acrylate und C₄-C₁₄-Cycloalkyl(meth)acrylate, besonders bevorzugt lineare C₂-C₁₆-Alkyl- und C₆-C₁₀-Cycloalkylmethacrylate und ganz besonders bevorzugt lineare C₂-C₁₀-Alkylmethacrylate, insbesondere n-Propylmethacrylat (T_{G} = 35°C), Neopentylmethacrylat, n-Butylmethacrylat (T_{G} = 20°C), Phenylethylmethacrylat (T_{G} = 26°C), n-Pentylmethacrylat, n-Hexylmethacrylat (T_{G} = -5°C), Cyclohexylmethacrylat, 2-Ethylhexylmethacrylat, n-Octylmethacrylat (T_{G} = -20°C), n-Dodecylmethacrylat (T_{G} = -65°C), Tetradecylmethacrylat (T_{G} = -9°C) und Hexadecylmethacrylat (T_{G} = 16°C) sowie die entsprechende Acrylate und Mischungen davon, wobei in allen Fällen die Methacrylate gegenüber den Acrylaten bevorzugt sind. Am meisten bevorzugt sind n-Butylmethacrylat, n-Hexylmethacrylat, 2-Ethylhexylmethacrylat und Mischungen davon.

Bevorzugte **OH-Gruppen-haltige (Meth)acrylate M_{F}** sind monofunktionelle, radikalisch polymerisationsfähige Monomere, besonders bevorzugt monofunktionelle Monomere mit einer (1) OH-Gruppe. Ganz besonders bevorzugt sind 2-Hydroxyethyl(meth)acrylat, 2-Hydroxypropyl(meth)acrylat, 3-Hydroxypropyl(meth)-acrylat, 4-Hydroxybutyl(meth)acrylat und Mischungen davon. Ganz besonders bevorzugt sind 2- und 3-Hydroxypropylmethacrylat (HPMA), insbesondere 2-Hydroxyethylmethacrylat (HEMA) und Mischungen davon.

Die Hydroxylgruppe der Monomere M_{F} gestattet eine polymeranaloge Funktionalisierung des Copolymers A₀ mit polymerisationsfähigen **Monomeren M_{P}**, die eine (1) funktionelle Gruppe, die mit der Hydroxylgruppe unter Ausbildung einer chemischen Bindung reagieren können, und mindestens eine radikalisch polymerisierbare Gruppe tragen. Bevorzugte funktionelle Gruppen sind Isocyanatgruppen, bevorzugte radikalisch polymerisierbare Gruppen sind (Meth)acrylatgruppen und insbesondere Methacrylatgruppen.

Bevorzugte Monomere M_{P} sind 2-Isocyanatoethyl(meth)acrylat, die Additionsprodukte von 1 Mol 2-Hydroxyethyl(meth)acrylat oder 2-Hydroxypropyl(meth)acrylat mit jeweils 1 Mol eines Diisocyanats, vorzugsweise Hexamethylen-1,6-diisocyanat (HMDI), 2,2,4-Trimethyhexamethylen-1,6-diisocyanat (TMDI) oder Isophorondiisocyanat (IPDI) und Mischungen davon. Ein besonders bevorzugtes Monomer M_{P} ist 2-Isocyanatoethylmethacrylat (IEMA).

Durch den Gehalt der Monomerbausteine M_{F} im Copolymer A₀ und den Umsetzungsgrad der polymeranalogen Reaktion mit dem oder den Monomeren M_{P} kann der Gehalt an polymerisationsfähigen Gruppen im Copolymer A_{P} und damit der Vernetzungsgrad gezielt eingestellt werden. Vorzugsweise wird die Menge an Monomeren M_{P} so gewählt, dass 10 bis 100 Mol-%, bevorzugt 30 bis 100 Mol-% und besonders bevorzugt 40 bis 100 Mol-% der OH-Gruppen der Monomerbausteine M_{F} mit dem Monomer M_{P} umgesetzt werden.

Stark saure **Haftmonomere M_{H}** sind radikalisch polymerisationsfähige Monomere, die mindestens eine stark saure Gruppe und eine (1) radikalisch polymerisierbare Gruppe tragen. Bevorzugte Haftmonomere M_{H} sind Monomere mit einem pKₐ-Wert (bei Raumtemperatur) von 0,5 bis 4,0, vorzugsweise 1,0 bis 3,5 und besonders bevorzugt 1,5 bis 2,5. Bevorzugte stark saure Gruppen sind Phosphorsäure- und Phosphonsäuregruppen, bevorzugte radikalisch polymerisierbare Gruppen sind (Meth)acrylatgruppen und insbesondere Methacrylatgruppen. Besonders bevorzugte Haftmonomere M_{H} sind radikalisch polymerisationsfähige Phosphorsäureester, wie 2-(Meth)acryloyloxypropyldihydrogenphosphat, 2-(Meth)acryloyloxyethyldihydrogen-phosphat, 4-(Meth)acryloyloxybutyldihydrogenphosphat, 6-(Meth)acryloyloxyhexyldi-hydrogenphosphat, 10-Acryloyloxydecyldihydrogenphosphat (ADP) und 10-Methacryloyloxydecyldihydrogenphosphat (MDP) sowie polymerisationsfähige Phosphonsäuren, wie Vinylphosphonsäure, 4-Vinylphenylphosphonsäure, 4-Vinylbenzylphosphonsäure, 2-(Meth)acryloyloxyethylphosphonsäure, 2-[4-(Dihydroxyphos-phoryl)-2-oxa-butyl]-acrylsäureethylester (DHPBAE) und Mischungen davon. Ganz besonders bevorzugt sind MDP, Vinylphosphonsäure, DHPBAE und Mischungen davon.

Unter radikalisch polymerisationsfähigen **Carbonsäure-Monomere M_{CS}** werden Monomere mit mindestens einer Carbonsäuregruppe, vorzugsweise 1 bis 2 Carbonsäuregruppen, und einer (1) radikalisch polymerisierbaren Gruppe verstanden. Bevorzugte radikalisch polymerisierbar Gruppen sind Vinyl-, Acryl- sowie vicinal oder geminal substituierte Ethylengruppen und insbesondere Methacrylgruppen. Ethylengruppen können beispielsweise durch -CH₃ und -COOH, -COOH und -CH₂-COOH oder zwei COOH-Gruppen vicinal oder geminal substituiert sein. Bevorzugte Monomere M_{CS} sind (Meth)acrylsäure, Itaconsäure, Crotonsäure, Maleinsäure und Fumarsäure sowie deren Halbester, 2-(Hydroxymethyl)acrylsäure, 4-(Meth)acryloyl-oxyethyltrimellitsäure, 10-(Meth)acryloyloxydecylmalonsäure, 2-(Meth)acryloyloxy-methylbernsteinsäure, 4-Vinylbenzoesäure und Mischungen davon. Besonders bevorzugt sind Acrylsäure und insbesondere Methacrylsäure (MAA).

Erfindungsgemäß bevorzugt sind Adhäsive, die neben dem Copolymer A_{P} mindestens ein zweites **Copolymer B_{P}** enthalten, dessen Zusammensetzung sich von der des Copolymers A_{P} unterscheidet, vorzugsweise ein Copolymer, das erhältlich ist durch
(I) Copolymerisation von
   (a) einem oder mehreren Monomeren M_{G} mit einer (1) polymerisationsfähigen (Meth)acrylatgruppe,
   (b) einem oder mehreren OH-Gruppen-haltigen (Meth)acrylaten M_{F}, und
   (c) ggf. einem oder mehreren polymerisationsfähigen Carbonsäure-Monomeren M_{CS},
(II) Umsetzen des erhaltenen Copolymers B₀ in einer polymeranalogen Reaktion mit mindestens einem funktionalisierten, radikalisch polymerisationsfähigen Monomer M_{P}, das mit den OH-Gruppen des Monomers M_{F} in der Polymerkette von B₀ reagiert und dabei ein Copolymer B_{P} bildet, das seitenständige polymerisationsfähige Gruppen trägt, und
(III) Neutralisieren des Copolymers Bₚ mit einer Base.

Das Copolymer B₀ weist vorzugsweise die folgende Zusammensetzung auf:
(a) 30 bis 85 Gew.-%, besonders bevorzugt 40 bis 80 Gew.-% Monomer M_{G},
(b) 5 bis 55 Gew.-%, besonders bevorzugt 10 bis 50 Gew.-% Monomer M_{F} und
(c) 1 bis 40 Gew.-%, besonders bevorzugt 5 bis 30 Gew.-% Monomer M_{CS},
wobei sich alle Prozentangaben auf die Masse des Copolymers B₀ beziehen. Besonders bevorzugt sind Copolymere, bei denen die Summe von (a), (b) und (c) 100% beträgt.

Das Copolymer Bₚ wird vorzugsweise ebenfalls in Form einer Sekundärdispersion hergestellt. Die Herstellung der Sekundärdispersion von Copolymer B₀ erfolgt analog zu Copolymer A₀ durch Copolymerisation der Monomere M_{G}, M_{F} und ggf. M_{CS} in einem organischen Lösungsmittel (Schritt (I)). Das hierbei erhaltene Copolymer B₀ wird dann in einer polymeranalogen Reaktion mit dem Monomer Mₚ zu dem Copolymer B_{P} umgesetzt (Schritt (II)). Die Menge an Monomeren M_{P} wird vorzugsweise so gewählt, dass 10 bis 100 Mol-%, bevorzugt 30 bis 80 Mol-% und besonders bevorzugt 40 bis 60 Mol-% der OH-Gruppen der Monomerbausteine M_{F} mit einem Monomer M_{P} reagieren. Anschließend kann das Copolymer B durch Zugabe einer Base neutralisiert (Schritt (III)), die erhaltene Copolymer-Lösung ggf. mit Wasser versetzt (Schritt (IV)) und dann das organische Lösungsmittel ggf. abdestilliert werden (Schritt (V)). Die erhaltene Sekundärdispersion des Copolymers B_{P} kann anschließend mit einer Sekundärdispersion des Copolymers A_{P} gemischt werden. Sekundärdispersionen, die zwei oder mehr unterschiedliche Copolymere enthalten, werden hierin als Sekundärcodispersionen bezeichnet. Zur Herstellung von Copolymer B_{P} werden vorzugsweise die oben beschriebenen Monomere M_{G}, M_{F}, M_{P} und ggf. M_{CS} verwendet, wobei zur Herstellung B_{P} dieselben oder andere Monomere wie zur Herstellung von A_{P} eingesetzt werden können. Das Copolymer B_{P} enthält keine stark sauren Monomere M_{H}, bzw. davon abgeleitete Wiederholungseinheiten. Zur Herstellung des Copolymers B_{P} werden in allen Fällen ausschließlich monofunktionelle Monomere, d.h. Monomere mit einer radikalisch polymerisationsfähigen Gruppe, eingesetzt.

Gemäß einer bevorzugten Ausführungsform der Erfindung werden die Sekundärcodispersionen hergestellt, indem die Lösungen der Copolymere A_{P} und B_{P} separat hergestellt und vor der Neutralisation miteinander gemischt und erst danach durch Zugabe einer Base neutralisiert werden. Anschließend wird die neutralisierte Copolymerdispersion ggf. mit Wasser versetzt und dann das organische Lösungsmittel ggf. abdestilliert.

Bevorzugte **Basen** zur Neutralisation der sauren Monomerbausteine der Copolymere Aₚ, B_{P} bzw. der Mischung aus A_{P} und B_{P} sind LiOH, KOH, NaOH, NH₄OH und organische Amine, vorzugsweise Ethylamin, n-Butylamin, Dibutylamin, Trimethylamin, Tributylamin (TBA), Triethylamin (TEA), N-Methylmorpholin, oder Dimethylcyclohexylamin, Aminosäurederivate, vorzugsweise Lysinmethylesterdihydrochlorid, Argininhydrochlorid oder Glycinethylester, OH-funktionalisierte Amine, vorzugsweise Methyldiethanolamin, Diethylethanolamin, Dimethylethanolamin, Tris(hydroxymethyl)aminomethan, Ethanolamin, Diethanolamin, Triethanolamin (TEOA), Butanolamin, Dibutanolamin, 3-Dimethylamino-2-propanol (DMAP) oder 1,3-Diamino-2-propanol (DAP), höhermolekulare ethergruppenhaltige Amine und Mischungen davon. Bevorzugte höhermolekulare ethergruppenhaltige Amine sind Aminogruppenterminierte oligomere Ethylenoxid- oder Propylenoxid-Oligomere. Solche Polyetheramine sind z.B. unter der Bezeichnung JEFFAMINE^{®} kommerziell verfügbar. Die genannten Basen werden vorzugsweise in Form wässriger Lösungen eingesetzt. Besonders bevorzugt sind wässrige Lösungen von DMAP, TBA, TEA, DAP und Mischungen davon.

Die sauren Gruppen der Copolymere A_{P} und B_{P} können ganz oder vorzugsweise teilweise neutralisiert werden. Vorzugsweise wird die Base oder Basenmischung in einer Menge von 20 bis 90 mol-%, bevorzugt von 25 bis 80 mol-% und besonders bevorzugt von 30 bis 75 mol-% bezogen auf den Gehalt an stark sauren Haftmonomeren M_{H} zugesetzt. Bei der Verwendung einer Mischung von A_{P} und B_{P} erfolgt die Neutralisation vorzugsweise nach dem Mischen der Copolymere.

Durch die Art der eingesetzten Basen und den Grad der Neutralisation kann die Partikelgröße der Copolymerpartikel in der gebildeten Sekundärdispersion gezielt eingestellt werden. Erfindungsgemäß sind Partikel mit einer mittleren Partikelgröße von 1 bis 700 nm, insbesondere von 5 bis 500 nm und ganz besonders von 20 bis 300 nm bevorzugt.

Wenn nicht anders angegeben, handelt es sich bei allen Partikelgrößen hierin um volumengemittelte Partikelgrößen (D50-Werte), d.h. 50 % des Gesamtvolumens, das alle Partikel gemeinsam besitzen, ist in Partikeln enthalten, die einen Durchmesser kleiner als der angegebene Wert aufweisen. Der D10-Wert ist dementsprechend der volumetrische Durchmesser bei dem 10% des gesamten Füllstoffvolumens kleiner als der genannte Wert sind.

Die Partikelgrößenbestimmung im Bereich von 0,1 µm bis 1000 µm erfolgt vorzugsweise mittels statischer Lichtstreuung (SLS), beispielsweise mit einem statischen Laserstreuungs-Partikelgrößen-Analysator LA-960 (Horiba, Japan) oder mit einem Microtrac S100 Partikelgrößen-Analysator (Microtrac, USA). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße in einem Bereich von 1 nm bis 0,1 µm erfolgt vorzugsweise durch dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Im Fall aggregierter und agglomerierter Partikel kann die Primärteilchengröße anhand von TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf ein 50 Å dickes Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Die erfindungsgemäßen **Adhäsive** enthalten neben dem oder den Copolymeren A_{P} und ggf. B_{P} vorzugsweise mindestens ein Dispersionsmittel, besonders bevorzugt Ethanol, Isopropanol, tert.-Butylalkohol, Wasser oder eine Mischung davon, ganz besonders bevorzugt Ethanol, Wasser oder eine Mischungen davon. Am meisten bevorzugt ist Wasser.

Weiterhin können die Adhäsive einen oder mehrere Initiatoren für die radikalische Polymerisation enthalten, vorzugsweise einen Photoinitiator, insbesondere Campherchinon in Kombination mit 4-Dimethylaminobenzoesäureethylester. Die Polymerisation der Adhäsive kann aber beispielsweise auch zusammen mit der Polymerisation eines radikalisch aushärtbaren Restaurationsmaterials erfolgen, das auf das Adhäsiv aufgebracht wird. Es wird angenommen, dass hierbei Initiatorbestandteile und/oder Radikale aus dem Restaurationsmaterial in die Adhäsivschicht diffundieren und eine Härtung des Adhäsivs induzieren. Die Zugabe eines Initiators ist daher optional, und Adhäsive, die keinen Initiator für die radikalische Polymerisation enthalten, sind besonders bevorzugt.

Außerdem können die Adhäsive einen oder mehrere Füllstoffe und/oder ein oder mehrere Additive enthalten.

Vorzugsweise haben die erfindungsgemäßen Adhäsive die folgende Zusammensetzung:
- 10 bis 50 Gew.-%, bevorzugt 15 bis 45 Gew.-% und besonders bevorzugt 20 bis 35 Gew.-% mindestens eines Copolymers A_{P} und ggf. mindestens eines Copolymers B_{P} und
- 50 bis 90 Gew.-%, bevorzugt 55 bis 85 Gew.-% und besonders bevorzugt 65 bis 80 Gew.-% Dispersionsmittel.

Die Adhäsive enthalten vorzugsweise
- 10 bis 70 Gew.-%, vorzugsweise 15 bis 60 Gew.-% und besonders bevorzugt 20 bis 55 Gew.-% Wasser.

Sie können außerdem
- von 0,001 bis 10 Gew.-%, bevorzugt 0,01 bis 8 Gew.-% und besonders bevorzugt 0,1 bis 5 Gew.-% Initiator für die radikalische Polymerisation enthalten.

Weiter bevorzugt sind Adhäsive, die
- 0 bis 10 Gew.-%, bevorzugt 0 bis 5 Gew.-% und besonders bevorzugt 0 bis 3 Gew.-% Füllstoff und/oder
- 0 bis 5 Gew.-%, bevorzugt 0 bis 3 Gew.-% und besonders bevorzugt 0 bis 2 Gew.-% eines oder mehrerer Additive enthalten.

Alle Mengenangaben beziehen sich jeweils auf die Gesamtmasse des Adhäsivs, wobei der Wassergehalt ggf. auf die Dispersionsmittelmenge angerechnet wird. Die erfindungsgemäßen Adhäsive liegen vorzugweise in einkomponentiger Form vor.

Die Menge des Dispersionsmittels wird so bemessen, dass die Adhäsive die gewünschte Viskosität aufweisen, d.h. leicht applizierbar sind und auf dem zu verklebenden Substrat, z.B. dem Zahn, gut verstrichen werden können.

Bevorzugte **Initiatoren** zur Herstellung der Adhäsive sind Photoinitiatoren wie Benzophenon, und Benzoin sowie deren Derivate, sowie α-Diketone und deren Derivate, wie 9,10-Phenanthrenchinon, 1-Phenyl-propan-1,2-dion, Diacetyl oder 4,4'-Dichlorbenzil. Bevorzugte Photoinitiatoren sind Campherchinon und 2,2-Dimethoxy-2-phenyl-acetophenon und insbesondere α-Diketone in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin. Bevorzugt sind auch Norrish-Typ-I-Photoinitiatoren, insbesondere Acyl- der Bisacylphosphinoxide, Monoacyltrialkyl- und Diacyldialkylgermanium-Verbindungen, wie z.B. Benzoyltrimethylgermanium, Dibenzoyldiethylgermanium und Bis(4-methoxybenzoyl)diethylgermanium. Weiter bevorzugt sind Mischungen verschiedener Photoinitiatoren, wie z.B. Dibenzoyldiethylgermanium in Kombination mit Campherchinon und 4-Dimethylaminobenzoesäureethylester. Am meisten bevorzugt ist Campherchinon in Kombination mit 4-Dimethylaminobenzoesäureethylester.

Bevorzugte **Füllstoffe** sind organische oder anorganische Füllstoffpartikel. Bevorzugte anorganische partikuläre Füllstoffe sind amorphe kugelförmige Materialien auf der Basis von Oxiden, wie SiO₂ oder ZrO₂, oder Mischoxiden aus SiO₂ und ZrO₂. Nanopartikuläre Füllstoffe, wie pyrogene Kieselsäure oder Fällungskieselsäure mit einer durchschnittlichen Primärteilchengröße von 5 bis 200 nm und vorzugsweise von 10 bis 50 nm sind besonders bevorzugt. Füllstoffe dienen zur Verbesserung der mechanischen Eigenschaften und/oder zur Einstellung der Viskosität und werden vorzugsweise vor der Bildung der Dispersion zugegeben werden. Die Adhäsive können einen oder mehrere Füllstoffe enthalten.

Als weitere **Additive** eignen sich vor allem Stabilisatoren, mikrobizide Wirkstoffe und fluoridionenabgebende Additive.

Es wurde überraschend gefunden, dass die erfindungsgemäßen Adhäsive eine hohe Haftung auf der natürlichen Zahnhartsubstanz aufweisen und so eine sichere Befestigung direkter und indirekter Restaurationen an Zähnen ermöglichen. Sie eignen sich darüber hinaus aber auch zum Verkleben von Restaurationen miteinander und zur Reparatur von Dentalrestaurationen. Die Copolymere A_{P} und B_{P} tragen radikalisch polymerisierbare Gruppen, die eine Vernetzung und Härtung der Adhäsive ermöglichen und gute mechanische Eigenschaften gewährleisten. Außerdem können diese Gruppen mit radikalisch polymerisierbaren Gruppen beispielsweise eines dentalen Füllungsmaterials reagieren und so dessen Haftung verbessern.

Hervorzuheben ist, dass die Adhäsive auch unter oralen Bedingungen eine hohe und dauerhafte Haftung aufweisen. Dies ist besonders überraschend, weil sowohl das Copolymer A_{P} als auch das Copolymer B_{P} zu einem erheblichen Anteil aus hydrophilen Monomeren aufgebaut sind, so dass die daraus gebildeten Haftschichten hydrophil und damit feuchtigkeitsempfindlich sein sollten. Die Erfinder haben gefunden, dass durch eine gezielte Auswahl von Art und Menge der zur Herstellung der Copolymere A_{P} und B_{P} verwendeten Monomere und durch den gezielten Einbau seitenständiger polymerisationsfähiger Gruppen in die Copolymerketten Adhäsive erhalten werden können, die auch unter feuchten Bedingungen eine stabile Haftung gewährleisten und damit für dentale Anwendungen und insbesondere für die intraorale Anwendung hervorragend geeignet sind.

Ein besonderer Vorteil der erfindungsgemäßen Adhäsive ist, dass sie keine Monomere und insbesondere kein radikalisch polymerisierbaren Monomere enthalten, was unter toxikologischen Gesichtspunkten vorteilhaft ist. Die Adhäsive zeichnen sich daher durch eine hohe Biokompatibilität aus. Darüber hinaus enthalten die Adhäsive vorzugsweise auch keine Emulgatoren und Tenside.

Die **Biokompatibilität** wurde mit standardisierten Testverfahren anhand wässriger Extrakte der gehärteten Adhäsive untersucht. Die Extrakte zeigten keine Cytotoxizität und keine Genotoxizität gegenüber Mausfibroblasten.

Im Folgenden wird ein bevorzugtes Verfahren zur Herstellung der erfindungsgemäßen Copolymere beschrieben.

Die **Synthese der Copolymere A_{P} und B_{P}** erfolgt durch radikalische Polymerisation der Monomeren M_{G}, M_{F}, und ggf. M_{H} und M_{CS},vorzugsweise durch Lösungspolymerisation in einem leicht flüchtigen, mit Wasser mischbaren organischen Lösungsmittel, das Poly(meth)acrylate gut löst. Bevorzugte Lösungsmittel sind Aceton (Siedepunkt: 56°C), Methylethylketon (80°C), Diethylketon (102°C), Methylisobutylketon (116°C), Tetrahydrofuran (66°C) und Acetonitril (82°C) und Mischungen davon. Besonders bevorzugt sind Aceton und Methylethylketon. Die Monomere werden dabei vorzugsweise in einer Gesamtmonomerkonzentration von 10 bis 60 Gew.-%, bevorzugt 15 bis 50 Gew.-% und besonders bevorzugt 20 bis 40 Gew.-%, bezogen auf die Masse des Reaktionsansatzes, eingesetzt.

Die Polymerisation wird vorzugsweise in Anwesenheit eines Initiators für die radikalische Polymerisation durchgeführt. Bevorzugte Polymerisationsinitiatoren zur Herstellung der Copolymere sind thermische Initiatoren wie Azoverbindungen, z.B. 2,2'-Azobis(isobutyronitril) (AIBN), 2,2'-Azobis(2-methylbutyronitril), 4,4'-Azobis(4-cyanvaleriansäure), Dimethyl-2,2'-azobisbutyrat, 1,1'-Azobis(1-cyclohexancarbonitril) oder 2,2'-Azobis(2,4-dimethyl-4-methoxyvaleronitril), Peroxide, z.B. Dilaurylperoxid, tert.-Butylperoxy-2-ethylhexanoat, tert.-Butylperoxybenzoat oder Di-tert.-butylperoxid, Dicumylperoxid, Peroxidicarbonate, z.B. Dicyclohexylperoxidicarbonat oder Bis-(4-tert-butylcyclohexyl)peroxidicarbonat. Besonders bevorzugt sind AIBN, tert.-Butylperoxy-2-ethylhexanoat, tert.-Butylperoxybenzoat oder Di-tert.butylperoxid. Um einen vollständigen Monomerumsatz zu erreichen, ist es bevorzugt, den Initiator im Verlaufe der Polymerisation nachzudosieren. Gegebenenfalls kann dabei bei erhöhter Temperatur nachpolymerisiert werden. Die insgesamt verwendete Initiatorkonzentration liegt vorzugsweise im Bereich von 0,1 bis 5,0 Gew.-%, besonders bevorzugt 0,5 bis 4,0 Gew.-% und ganz besonders bevorzugt von 0,7 bis 2,5 Gew.-% bezogen auf die Masse des Reaktionsansatzes. Eventuell vorhandene Restmonomere werden vorzugsweise durch Dialyse oder Umfällen der Copolymere abgetrennt.

Die **zahlenmittlere Molmasse Mₙ** der gebildeten Polymere nimmt mit zunehmender Monomerkonzentration und abnehmender Initiatorkonzentration zu. Zur gezielten Einstellung der Molmasse, auch bei hohen Monomerkonzentrationen, kann ein Kettenregler zugesetzt werden. Bevorzugte Kettenregler sind Thiole, z.B. Mercaptoethanol, tert.-Dodecylmercaptan oder Laurylmercaptan, Disulfide, wie z.B. Diisopropylxanthogendisulfid. Besonders bevorzugte Kettenregler sind Thiolgruppen enthaltende Aminosäurederivate wie N-Acetyl-(L)-cystein (AC), N-Acetyl-L-cysteinmethylester, N-Acetyl-L-cysteinethylester, ganz besonders bevorzugt ist AC. Thiolgruppen enthaltende Aminosäurederivate ergeben Polymere mit besonders guter Biokompatibilität. Kettenregler werden vorzugsweise in einer Konzentration von 0,1 bis 5,0 Gew.-%, bevorzugt 0,5 bis 4,0 Gew.-% und besonders bevorzugt von 0,7 bis 3,0 Gew.-%, bezogen auf die Masse des Reaktionsansatzes, eingesetzt.

Initiatoren und Kettenregler werden bei der Polymerisation als Endgruppen in das Polymer eingebaut. Wie in der Polymerchemie üblich, werden diese bei Angabe der Polymerzusammensetzung nicht berücksichtigt.

Die zahlenmittlere Molmasse Mₙ der Copolymere A₀ und B₀ liegt vorzugsweise in einem Bereich von 5.000 bis 60.000 g/mol, besonders bevorzugt von 10.000 bis 50.000 g/mol und ganz besonders bevorzugt von 15.000 bis 40.000 g/mol, wobei die zahlenmittlere Molmasse Mₙ mittels Gelpermeationschromatographie (GPC) bestimmt wird.

Die Gelpermeationschromatographie (GPC) ist eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards. Als Kalibrierstandard werden vorzugsweise engverteile Polystyrolstandards eingesetzt. Diese sind kommerziell erhältlich. Als Trennmaterial werden Styrol-Divinylbenzol-Säulen und Tetrahydrofuran (THF) als Elutionsmittel verwendet. Styrol-Divinylbenzol-Säulen eignen sich für organische lösliche synthetische Polymere. Die Messung erfolgt mit verdünnten Lösungen (0,05 - 0,2 Gew.-%) der zu untersuchenden Polymere.

Alternativ kann die zahlenmittlere Molmasse mit den bekannten Methoden der Gefrierpunktserniedrigung (Kryoskopie), der Siedepunktserhöhung (Ebullioskopie) oder aus der Erniedrigung des Dampfdrucks (Dampfdruckosmometrie) bestimmt werden. Hierbei handelt es sich um absolute Methoden, die keine Kalibrierstandards erfordern. Es werden Konzentrationsreihen von 4 bis 6 verdünnten Polymerlösungen mit Konzentrationen von 0,005 bis 0,10 mol/kg untersucht und dann die gemessenen Werte auf eine Konzentration von 0 mol/kg extrapoliert.

Anschließend werden die Copolymere in einer polymeranalogen Reaktion mit dem Monomer M_{P} umgesetzt, vorzugsweise mit 2-Isocyanatoethylmethacrylat (IEMA). Die Reaktion wird vorzugsweise in der Copolymerlösung aus dem ersten Schritt durchgeführt. Dazu wird das Monomer Mₚ, z.B. IEMA, in einem organischen Lösungsmittel, vorzugsweise in Aceton, Methylethylketon, Diethylketon, Methylisobutylketon, Tetrahydrofuran oder Acetonitril, besonders bevorzugt in Aceton, gelöst und bei einer Temperatur von 20 bis 60°C, bevorzugt 40 bis 55°C, zu der Polymerlösung getropft. Zur Beschleunigung der Reaktionen können Katalysatoren, wie z.B. Zinn(II)-oxalat oder -octanoat. Dibutylzinndilaurat, Tri-o-tolyl-bismut, Triphenylbismut, Tris-(2-methylphenyl)-bismut, Tris-(2-methoxyphenyl)-bismut oder Bismut(III)-neodecanoat zugesetzt werden. Besonders bevorzugt sind Bismutkatalysatoren, wie Tri-o-tolyl-bismut oder Bismut(III)-neodecanoat, die sich durch eine hohe Biokompatibilität auszeichnen. Gemäß einer ganz besonders bevorzugten Ausführungsform wird die Umsetzung mit dem Monomer M_{P} ohne Katalysator durchgeführt.

Anschließend werden die sauren Gruppen der Copolymere Aₚ und B_{P} durch Zugabe einer Base neutralisiert. Zur Herstellung von Sekundärcodispersionen werden die Copolymere A_{P} und B_{P} vorzugsweise vor der Neutralisation miteinander gemischt. Das Copolymer Aₚ bzw. die Mischung aus A_{P} und B_{P} wird, gelöst in einem mit Wasser zumindest teilweise mischbaren organischen Lösungsmittel, das eine geringere Siedetemperatur als Wasser hat, oder in einem Azeotrop, dessen Siedepunkt niedriger als der von Wasser ist, mit einer oder mehreren Basen versetzt. Vorzugsweise wird die Base in Form einer wässrigen Lösung schrittweise zugegeben.

Wenn die Wassermenge der Basenlösung nicht zum Dispergieren der Copolymerlösung ausreicht oder wenn die Base nicht in Form einer wässrigen Lösung eingesetzt wird, wird die neutralisierte Copolymerlösung mit Wasser versetzt. Die Mischung wird vorzugsweise intensiv gerührt und das organische Lösungsmittel vorzugsweise anschließend zumindest teilweise abgedampft. Beim Verdampfen des Lösungsmittels bildet sich eine viskose Sekundärdispersion oder Sekundärcodispersion. Die erhaltene Sekundärdispersion oder Sekundärcodispersion enthält Partikel des oder der Copolymere. Die Sekundärdispersion oder Sekundärcodispersion kann direkt als Adhäsiv oder zur Herstellung des Adhäsivs verwendet werden.

Die vorteilhaften Eigenschaften der erfindungsgemäßen Dentalwerkstoffe sind großenteils auf die radikalisch polymerisationsfähigen Copolymere A_{P} und ggf. B_{P} zurückzuführen. Diese liegen in der Form von Partikeln vor, die die Herstellung stabiler Dispersionen ermöglichen. Die Copolymere tragen saure Gruppen, die nach der Neutralisation eine anionische Stabilisierung der Dispersionen bewirken. Gleichzeitig wird durch die Kombination von stark sauren Monomeren und Carbonsäure-Monomeren eine hohe Haftung an Dentin und Zahnschmelz erzielt. Nach der Applikation der Adhäsive auf die Zahnoberfläche und anschließendem Trocken bilden die Copolymere einen gut haftenden Polymerfilm, der über die polymerisationsfähigen Gruppen des Monomers M_{P} einen festen Verbund mit dentalen Restaurationsmaterialien wie z.B. Füllungskompositen ermöglicht. Die Copolymere A_{P} und ggf. B_{P} ermöglichen die Herstellung von einkomponentigen, monomerfreien Adhäsiven mit hoher Biokompatibilität.

Erfindungsgemäß geeignete Copolymere lassen sich vorzugsweise auf die oben beschriebene Weiser herstellen. Die Synthese der Copolymere A_{P} und B_{P} kann aber auch durch andere bekannte Verfahren der radikalischen Polymerisation erfolgen, beispielsweise durch Substanzpolymerisation, Fällungspolymerisation, Suspensions- oder Emulsionspolymerisation sowie die im Stand der Technik bekannten Verfahren der lebenden radikalischen Polymerisation. Die hierbei erhaltenen Copolymere können in einem mit Wasser zumindest teilweise mischbaren organischen Lösungsmittel mit einer Siedetemperatur unter der von Wasser gelöst und durch polymeranaloge Reaktion mit einem polymerisationsfähigen Monomer M_{P} umgesetzt werden. Die Polymerlösung wird dann in Wasser unter starkem Rühren dispergiert, die Säuregruppen vor, nach oder während des Dispergierens in Wasser ganz oder teilweise mit einer Base neutralisiert und schließlich das organische Lösungsmittel weitgehend verdampft. Danach können die Copolymere in einem geeigneten Dispersionsmittel dispergiert werden.

Die erfindungsgemäßen Werkstoffe eignen sich besonders als Dentalwerkstoffe zur intraoralen Verwendung durch den Zahnarzt zur Restauration geschädigter Zähne (therapeutische Anwendung), ganz besonders als dentale Adhäsive und insbesondere als Schmelz-Dentin-Adhäsive.

Die erfindungsgemäßen Werkstoffe können auch als extraorale Werkstoffe (nicht therapeutisch) eingesetzt werden, beispielsweise als Adhäsive bei der Herstellung oder Reparatur von Dentalrestaurationen. Sie eignen sich zudem als Adhäsive zur Herstellung und Reparatur von Inlays, Onlays, Kronen oder Brücken.

Zur Anwendung werden die erfindungsgemäßen Adhäsive vorzugsweise mit einem Pinsel auf die präparierte Schmelz- und/oder Dentinoberfläche aufgetragen und evtl. darauf verrieben. Die Filmbildung kann durch Verdunsten der Flüssigkeit, z.B. durch Verblasen mit Luft oder durch Erwärmen mit einer künstlichen Wärmequelle oder Strahlung, beschleunigt werden. Hierbei bildet sich ein haftender Polymerfilm. Die Schmelz- und/oder Dentinoberfläche kann vorher durch übliche Maßnahmen, wie z. B. Ätzen mit 37 %-iger Phosphorsäure und/oder Trocknen mit einem Luftstrom, vorkonditioniert werden. Dies ist jedoch nicht erforderlich, und es wird auch ohne Säurevorbehandlung eine ausgezeichnete Haftung erzielt. Danach kann z.B. ein lichthärtendes Füllungskomposit appliziert und durch Bestrahlung mit Licht gehärtet werden. Enthält das Adhäsiv einen Photoinitiator, kann die Adhäsivschicht vor der Applikation des Füllungskomposits durch Bestrahlen gehärtet werden.

Mit den erfindungsgemäßen Adhäsiven können Scherhaftfestigkeiten auf Dentin und Schmelz von 15 MPa oder mehr erreicht werden, was einen sehr guten und dauerhaften Verbund von Füllungsmaterials und Zahnhartsubstanz gewährleistet.

Die Erfindung wird im Folgenden anhand von Beispielen näher erläutert. Die Beispiele belegen, dass die erfindungsgemäßen Schmelz-Dentin-Einkomponenten-Adhäsive biokompatibel sind und zu einer guten Schmelz-Dentin-Haftung führen.

### Ausführungsbeispiele

### Beispiel 1:

### Synthese von Copolymer A_{P} durch geregelte radikalische Copolymerisation in Lösung und Funktionalisierung mit 2-Isocyanatoethylmethacrylat (IEMA)

### a) Copolymerisation in Lösung:

In einem Becherglas wurden 55 g n-Butylmethacrylat (BuMA), 15 g 2-Hydroxyethylmethacrylat (HEMA) 20 g MDP, 10 g Methacrylsäure (MAA) und in 100 g Aceton unter Hilfe eines Ultraschallbades gelöst. In einem zweiten Becherglas wurden 1 g AIBN und 1 g *N*-Acetylcystein unter Rühren in 50 g Aceton gelöst. Die beiden Lösungen wurden vereinigt und in den großen Tropftrichter einer Apparatur, bestehend aus einem 500 ml Dreihalskolben versehen mit einem Rückflusskühler, 2 Tropftrichtern (50 ml und 250 ml), Stickstoffzufuhr (auf großem Tropftrichter), Blasenzähler (auf Rückflusskühler) und großem Rührfisch, überführt. In dem Dreihalskolben wurden 100 g Aceton vorgelegt. Anschließend wurden ca. 25 Vol.-% der Acrylatlösung in den Dreihalskolben eingelassen. Die gesamte Apparatur wurd für 15 Min. mit Stickstoff intensiv gespült. Danach wurde der Stickstofffluss so heruntergeregelt, dass eine Blase pro Sekunde im Blasenzähler sichtbar war. Unter Stickstoffstrom wurde die Lösung im Dreihalskolben für 30 Min. unter Rückfluss erhitzt (80°C Ölbadtemperatur), danach wurde die im Tropftrichter verbleibende Lösung binnen 90 Min. zur siedenden Polymerisationslösung zugetropft. Nach dem Zutropfen wurden über den kleinen Tropftrichter erneut 1 g AIBN gelöst in 50 g Aceton zügig zur siedenden Lösung zugetropft. Nach insgesamt 7 h Polymerisationszeit wurde die Lösungspolymerisation durch Ausschalten der Apparatur und Abkühlen beendet. Es wurde eine klare, leicht viskose Lösung der Copolymere **A₀** erhalten.

### b) Funktionalisierung von A₀ mit IEMA (= M_{P}) unter Bildung des Copolymers A_{P}

Die voranstehend erhaltene Polymerlösung von **A₀** wurde in einen 1-I-Dreihalskolben versehen mit einem 100 ml Tropftrichter und einem Rückflusskühler überführt. Es wurde eine Lösung aus 17,84 g IEMA gelöst in 50 g Aceton hergestellt und in den Tropftrichter überführt. Bei einer Temperatur von 50°C wurde die IEMA-Lösung binnen einer Stunde zur Polymerlösung zugetropft. Anschließend wurde die Lösung für weitere 3 h auf 50°C erhitzt. Zur Vervollständigung der Reaktion wurde die erhaltene Lösung anschließend für weitere 14 h bei Raumtemperatur gerührt. Die Kontrolle des vollständigen Umsatzes des Isocyanats erfolgte mittels FT IR-Spektroskopie (NCO-Bande). Die eingesetzte IEMA-Menge entsprach einer vollständigen Umsetzung der HEMA-Bausteine in **A₀**. Nach beendeter Reaktion war die Lösung sehr dickflüssig, so dass die Lösung des Copolymers **A_{P}** mit 100 g Aceton verdünnt wurde.

### Beispiel 2:

### Synthese von Copolymer B_{P} durch geregelte radikalische Copolymerisation in Lösung und Funktionalisierung mit IEMA

### a) Copolymerisation in Lösung:

In einem Becherglas wurden 60 g BuMA, 30 g HEMA und 10 g MAA in 100 Aceton gelöst. In einem zweiten Becherglas wurden 1 g AIBN und 1 g *N*-Acetylcystein unter Rühren in 50 g Aceton gelöst. Die beiden Lösungen wurden vereinigt und in den großen Tropftrichter einer Apparatur, bestehend aus einem 500 ml Dreihalskolben versehen mit einem Rückflusskühler, 2 Tropftrichtern (50 ml und 250 ml), Stickstoffzufuhr (auf großem Tropftrichter), Blasenzähler (auf Rückflusskühler) und großem Rührfisch, überführt. In dem Dreihalskolben wurden 100 g Aceton vorgelegt. Anschließend wurden ca. 25 Vol.-% der Methacrylatlösung in den Dreihalskolben eingelassen. Die gesamte Apparatur wurde für 15 Min. mit Stickstoff intensiv gespült. Danach wurde der Stickstofffluss so heruntergeregelt, dass eine Blase pro Sekunde im Blasenzähler sichtbar war. Unter Stickstoffstrom wurde die Lösung im Dreihalskolben für 30 Min. unter Rückfluss erhitzt (80°C Ölbadtemperatur), danach wurde die im Tropftrichter verbleibende Lösung binnen 90 Min. zur siedenden Polymerisationslösung zugetropft. Nach dem Zutropfen wurde über den kleinen Tropftrichter erneut 1 g AIBN gelöst in 50 g Aceton zügig zur siedenden Lösung zugetropft. Nach insgesamt 7 h Polymerisationszeit wurde die Lösungspolymerisation durch Ausschalten der Apparatur und Abkühlen beendet. Es wurde eine klare Lösung des Copolymers **B₀** erhalten.

### b) Funktionalisierung von B₀ mit IEMA (= M_{P}) unter Bildung des Copolymers B_{P}

Die voranstehend erhaltene Polymerlösung von **B₀** wurde in einen 1 l Dreihalskolben versehen mit einem 100 ml Tropftrichter und einem Rückflusskühler überführt. Es wurde eine Lösung aus 17,84 g IEMA gelöst in 50 g Aceton hergestellt und in den Tropftrichter überführt. Bei einer Temperatur von 50°C wurde die IEMA-Lösung binnen einer Stunde zur Polymerlösung zugetropft. Anschließend wurde die Lösung für weitere 18 h auf 50°C erhitzt. Die Kontrolle des vollständigen Umsatzes des Isocyanats erfolgte mittels FT IR-Spektroskopie (NCO-Bande). Die eingesetzte IEMA-Menge entsprach einer vollständigen Umsetzung der HEMA-Bausteine in **B₀**. Nach beendeter Reaktion wurde eine leicht gelbliche, klare Lösung der Copolymere **B_{P}** erhalten.

### Beispiel 3:

### Herstellung einer Sekundärcodispersion auf Basis von A_{P} und B_{P} (70% Neutralisation)

Etwa 1/8 der in Beispiel 1 erhaltenen Lösung von Copolymer **A_{P}** und etwa 1/8 der in Beispiel 2 erhaltenen Lösung von Copolymer **B_{P}** wurden separat in jeweils ein Becherglas eingewogen. Die beiden Lösungen wurden in einem 250 ml Rundkolben unter starkem Rühren gemischt. Die entstandene trübe Lösung wurde mit 560 mg Di-methylamino-1-propanol (DMAP) gelöst in 12,5 g demineralisiertem Wasser zügig mit einer Glaspipette in kleinen Portionen versetzt. Die DMAP-Menge entspricht der zur Neutralisation von 70% der Phosphorsäuregruppen in Copolymer A_{P} erforderlichen Menge. Nach beendeter Zugabe lag eine klare Lösung vor. Das Aceton wurde aus der Lösung am Rotationsverdampfer bei einem Druck von ca. 250 mbar und einer Wasserbadtemperatur von ca. 40°C entfernt. Nach dem Entfernen des organischen Lösungsmittels wurde eine Sekundärcodispersion als milchige, zähe Flüssigkeit erhalten.

### Beispiel 4:

### Herstellung eines Adhäsivs auf der Basis der Sekundärcodispersion aus Beispiel 3 und Messung der Scherhaftung an Zahnhartsubstanz

Zu 6 g der in Beispiel 3 erhaltenen Sekundärcodispersion wurden die Photoinitiatorkomponenten Campferchinon (CQ: 45 mg) und 4-(Dimethylamino)-benzoesäureethylester (EMBO: 16 mg), beide gelöst in zusammen 4 g Ethanol, gegeben und die Mischung durch intensives Schütteln zu einer klaren Lösung vermischt. Der Feststoffgehalt der Mischung lag bei 30 Gew.-%. Die Mischung wurde als Adhäsiv für Scherhaftmessungen eingesetzt.

Für Scherhaftmessungen auf Schmelz und Dentin wurden gemäß der ISO 29022: 2013 (Dentistry-Adhesion-Notched-edge shear bond strength test) Rinderzähne in einer Silikonform aus Dublisil 15 (additionsvernetzendes Vinyl-Polysiloxan, Firma Dreve) mit einem Einbettmittel/Giessharz (Visco-Voss GTS, Firma Vosschemie oder Bosworth Fast Tray), so eingebettet, dass sich die Labialseite des bovines Substrat auf der Unterseite/Boden der Silikonform befand und das bovine Substrat mit 2 bis 5mm Einbettmittel/Giessharz bedeckt war. Die mit Harz überschichteten Zähne wurden samt Form im Vakuumschrank bei ca. 400 mbar für ca. 30 s entlüftet. Die Aushärtung erfolgte über Nacht bei Raumtemperatur und -druck. Am nächsten Tag wurden die Prüfkörper aus der Silikonform entnommen. Die mit Schleifpapier (Körnung 120 und 400) geschliffenen Zahnoberflächen wurden mit handwarmem Wasser gespült und auf 37°C vortemperiert. Die Dentin- oder Schmelzoberflächen wurden trocken getupft (Kimtech Wipes). Alle Dentin- und Schmelz-Scherhaftfestigkeitswerte wurden ohne vorherige Ätzung der Zähne mit H₃PO₄ im sog. Selfetch-Modus bestimmt.

Anschließend wurde das Adhäsiv mittels Applikator (Microbrush-Applikator, Microbrush International, USA) ohne Säurevorbehandlung direkt auf die Zahnoberfläche appliziert, für ca. 20 s mit sanftem Druck agitiert und anschließend mit einem ölfreien Luftstrom zur Entfernung des Lösungsmittels so lange verblasen, bis ein unter dem Luftstrom unbeweglicher Film entstanden war. Danach wurde für 10 s mit einer LED-Lampe (Bluephase Style, 1200 mW/cm², 560 nm, Ivoclar Vivadent AG) belichtet. Der so vorbereitete Prüfkörper wurde mit der präparierten Seite nach oben in die Einspannvorrichtung nach EN ISO 29022: 2013 eingespannt. Es wurde darauf geachtet, dass beim Einbringen und Positionieren des Zahns, sowie beim Absetzen des Oberteils der Einspannvorrichtung die Adhäsivschicht nicht beschädigt wurde. Proben, bei denen die Adhäsivschicht durch vorzeitigen Kontakt mit dem Oberteil der Vorrichtung beschädigt wurde, wurden verworfen. Der Zahn wurde durch leichtes Anziehen der Halterungsschrauben in der Einspannvorrichtung fixiert. Anschließend wurde durch die Öffnung der Vorrichtung ein dentales Füllungskomposit (Tetric Evo-Ceram BulkFill, Ivoclar Vivadent AG) in einer Schichtstärke von 2 bis 4 mm appliziert. Der dabei aufgewendete Adaptions-Druck wurde so gewählt, dass das Komposit blasenfrei mit dem Adhäsiv in Kontakt gebracht wurde. Ein zu hoher Druck führt zum Austritt des Komposits unterhalb der Einspannvorrichtung und zur Bildung von Kompositwülsten um den Kompositzylinder herum. Prüfkörper mit solchen ringförmigen Kompositwülsten wurden verworfen. Anschließend wurde das Komposit entsprechend der Gebrauchsanweisung polymerisiert (10 s, 560 nm, Polymerisationslampe Bluephase Style, Ivoclar Vivadent AG).

Zur Messung der Scherhaftung wurden die adhäsiv befestigten Kompositzylinder mittels einer Zwick-Roell Z010 Prüfmaschine (500 N Messzelle, Prüfgeschwindigkeit 1 mm/min) in Anlehnung an die Ultradent-Methode (EN ISO 29022, Jahr 2013) bei 23°C vom Zahn abgeschert. Die Scherhaftfestigkeit wurde zu 16,2 MPa (Dentin) bzw. 19,5 MPa (Schmelz) bestimmt. Das sind für ein Schmelz-Dentin-Adhäsiv ausgezeichnete Haftverwerte, die für einen dauerhaften Verbund des Füllungskomposits mit der Zahnhartsubstanz gewährleisten.

### Beispiel 5:

### Messung der Biokompatibilität des Adhäsivs aus Beispiel 4

Zur Messung der Biokompatibilität des Adhäsivs aus Beispiel 4 wurde das Adhäsiv in dünnen Scheiben lichtgehärtet, und anschließend wurden die Scheiben in Wasser dispergiert. Die Zellvitalität wurde mit dem WST-1 Assay unter Verwendung von Mausfibroblasten der Zelllinie LS929 bestimmt. Bei diesem Testverfahren wird das Tetrazoliumsalz WST-1 (4-(3-(4-lodphenyl)-2-(4-nitrophenyl)-2H-5-tetrazolium)-1,3-benzol Disulfonat) eingesetzt, das von lebensfähigen, metabolisch aktiven Zellen enzymatisch zu einem roten Formazan-Farbstoff umgesetzt wird, dessen Farbintensität photometrisch bestimmt wird. Der Test wurde in Anlehnung an die Veröffentlichung von Y. M. Pupo et al. ("Cytotoxicity of Etch and-Rinse, Self-Etch, and Universal Dental Adhesive Systems in Fibroblast Cell Line 3T3" Scanning Vol. 2017, Article ID 9650420) durchgeführt. Als Positivkontrolle wurde monomeres HEMA verwendet. Es wurde keine Cytotoxizität gegenüber Mausfibroblasten gefunden.

Außerdem wurde die Genotoxizität in Anlehnung an die DIN EN ISO 10993-3 (2018) mittels COMET-Assay untersucht. Der Comet-Assay basiert auf einer gelelektrophoretischen Bestimmung geschädigter DNA-Stränge. Die Extrakte des gehärteten Adhäsivs zeigten im COMET-Assay keine DNA-schädigende Wirkungen und damit keine Genotoxizität gegenüber Mausfibroblasten.

### Beispiel 6:

### Herstellung einer Sekundärcodispersion auf Basis von A_{P} und B_{P} (50% und 30% Neutralisation)

Analog zu Beispiel 3 wurden jeweils 1/8 der in Beispiel 1 erhaltenen Lösung von Copolymer **A_{P}** und 1/8 der in Beispiel 2 erhaltenen Lösung von Copolymer **B_{P}** separat in jeweils ein Becherglas eingewogen. Die beiden Lösungen wurden in einem 250 ml Rundkolben unter starkem Rühren gemischt. Die entstandene trübe Lösung wurde mit 400 mg DMAP gelöst in 12,5 g demineralisiertem Wasser zügig mit einer Glaspipette in kleinen Portionen versetzt. In einem zweiten Ansatz wurde die entstandene trübe Lösung mit 240 mg DMAP gelöst in 12,5 g demineralisiertem Wasser versetzt. Die DMAP-Mengen entsprechen einer Neutralisation von 50% bzw. 30% der Phosphorsäuregruppen in Copolymer A_{P}. Das Aceton wurde aus den gebildeten, klaren Lösungen am Rotationsverdampfer bei einem Druck von ca. 250 mbar und einer Wasserbadtemperatur von ca. 40°C entfernt. Nach dem Entfernen des organischen Lösungsmittels wurde jeweils eine Sekundärcodispersion als milchige, dickflüssige Dispersionen erhalten.

Analog Beispiel 4 wurden mit diesen Dispersionen durch Zugabe von CQ, EMBO und Ethanol Adhäsive mit einem Feststoffgehalt von 30 % hergestellt und die Scherhaftung auf Zahnhartsubstanz untersucht. Für die zu 50% neutralisierte Dispersion wurden Haftwerte von 15,1 MPa (Dentin) bzw. 20,5 MPa (Schmelz) und für die zu 30% neutralisierte Dispersion Haftwerte von 18,1 MPa (Dentin) bzw. 21,9 MPa (Schmelz) gemessen.

## Patentansprüche

1. Dentalwerkstoff enthaltend mindestens ein Copolymer, **dadurch gekennzeichnet, dass** das Copolymer erhältlich ist durch
(I) Copolymerisation von
(a) einem oder mehreren Monomeren M_{G} mit einer polymerisationsfähigen (Meth)acrylatgruppe,
(b) einem oder mehreren OH-Gruppen-haltigen (Meth)acrylaten M_{F},
(c) einem oder mehreren stark sauren Haftmonomeren M_{H} und
(d) ggf. einem oder mehreren polymerisationsfähigen Carbonsäure-Monomere M_{CS},
(II) Umsetzen des in Schritt (I) erhaltenen Copolymers A₀ in einer polymeranalogen Reaktion mit mindestens einem funktionalisierten, radikalisch polymerisationsfähigen Monomer M_{P}, das mit den OH-Gruppen des Monomers M_{F} in der Polymerkette von A₀ reagiert und dabei ein Copolymer A_{P} bildet, das seitenständige polymerisationsfähige Gruppen trägt, und
(III) Neutralisieren des Copolymers Aₚ mit einer Base.

2. Dentalwerkstoff nach Anspruch 1, wobei das Copolymer A₀ die folgende Zusammensetzung aufweist:
(a) 20 bis 80 Gew.-%, besonders bevorzugt 40 bis 70 Gew.-% Monomer M_{G},
(b) 1 bis 70 Gew.-%, besonders bevorzugt 5 bis 40 Gew.-% Monomer M_{F},
(c) 1 bis 40 Gew.-%, besonders bevorzugt 5 bis 30 Gew.-% Monomer M_{H} und
(d) 0 bis 30 Gew.-%, besonders bevorzugt 5 bis 20 Gew.-% Monomer M_{CS},
wobei sich alle Prozentangaben auf die Masse des Copolymers A₀ beziehen.

3. Dentalwerkstoff nach Anspruch 1 oder 2, wobei das Copolymer Aₚ erhältlich ist, indem die Copolymerisation der Monomere M_{G}, M_{F}, M_{H} und ggf. M_{CS} zu Copolymer A₀ gemäß Schritt (I) in einem organischen Lösungsmittel durchgeführt wird, nach Umsetzung des Copolymers A₀ mit Monomer Mₚ (Schritt (II)) und anschließender Neutralisation (Schritt (III))
(IV) die in Schritt (III) erhaltenen Copolymer-Lösung ggf. mit Wasser versetzt und gerührt wird, und
(V) dann das organische Lösungsmittel ggf. abdestilliert wird.

4. Dentalwerkstoff nach einem der Ansprüche 1 bis 3, der neben dem Copolymer A_{P} zusätzlich ein zweites **Copolymer B_{P}** enthält, dessen Zusammensetzung sich von der des Copolymers A_{P} unterscheidet.

5. Dentalwerkstoff nach Anspruch 4, wobei das Copolymer B_{P} erhältlich ist durch
(I) Copolymerisation von
(a) einem oder mehreren Monomeren M_{G} mit einer polymerisationsfähigen (Meth)acrylatgruppe,
(b) einem oder mehreren OH-Gruppen-haltigen (Meth)acrylaten M_{F},
(c) ggf. einem oder mehreren polymerisationsfähigen Carbonsäure-Monomeren M_{CS},
(II) Umsetzen des erhaltenen Copolymers B₀ in einer polymeranalogen Reaktion mit mindestens einem funktionalisierten, radikalisch polymerisationsfähigen Monomer M_{P}, das mit den OH-Gruppen des Monomers M_{F} in der Polymerkette von B₀ reagiert und dabei ein Copolymer B_{P} bildet, das seitenständige polymerisationsfähige Gruppen trägt, und
(III) Neutralisieren des Copolymers Bₚ mit einer Base.

6. Dentalwerkstoff nach Anspruch 4 oder 5, wobei das Copolymer B₀ die folgende Zusammensetzung aufweist:
(a) 30 bis 85 Gew.-%, besonders bevorzugt 40 bis 80 Gew.-% Monomer M_{G},
(b) 5 bis 55 Gew.-%, besonders bevorzugt 10 bis 50 Gew.-% Monomer M_{F} und
(c) 1 bis 40 Gew.-%, besonders bevorzugt 5 bis 30 Gew.-% Monomer M_{CS},
wobei sich alle Prozentangaben auf die Masse des Copolymers B₀ beziehen.

7. Dentalwerkstoff nach einem der Ansprüche 4 bis 6, wobei die Mischung der Copolymere A_{P} und B_{P} erhältlich ist, idem
- man durch Copolymerisation der Monomere M_{G}, M_{F}, M_{H} und ggf. M_{CS} in einem organischen Lösungsmittel ein Copolymer A₀ herstellt und durch polymeranaloge Reaktion mit mindestens einem Monomer Mₚ in Copolymer A_{P} überführt,
- man durch Copolymerisation der Monomere M_{G}, M_{F} und ggf. M_{CS} in einem organischen Lösungsmittel ein Copolymer B₀ herstellt und durch polymeranaloge Reaktion mit mindestens einem Monomer Mₚ in Copolymer B_{P} überführt,
- man die Lösungen von Copolymer A_{P} und B_{P} miteinander mischt,
- man die Mischung durch Zugabe einer Base neutralisiert,
- man dann die neutralisierte Mischung ggf. mit Wasser versetzt und
- man dann ggf. das organische Lösungsmittel entfernt.

8. Dentalwerkstoff nach einem der Ansprüche 1 bis 7, bei dem Menge des Monomers M_{P} so bemessen ist, dass
10 bis 100 Mol-%, bevorzugt 30 bis 100 Mol-% und besonders bevorzugt 40 bis 100 Mol-% der OH-Gruppen der Monomerbausteine M_{F} in dem Copolymer A_{P} mit M_{P} umgesetzt sind und ggf.
10 bis 100 Mol-%, bevorzugt 30 bis 80 Mol-% und besonders bevorzugt 40 bis 60 Mol-% der OH-Gruppen der Monomerbausteine M_{F} in dem Copolymer B_{P} mit M_{P} umgesetzt sind.

9. Dentalwerkstoff nach einem der Ansprüche 1 bis 8, bei dem
das oder die Monomere M_{G} ausgewählt sind aus n-Propylmethacrylat, Neopentylmethacrylat, n-Butylmethacrylat, Phenylethylmethacrylat, n-Pentylmethacrylat, n-Hexylmethacrylat, Cyclohexylmethacrylat, 2-Ethylhexylmethacrylat, n-Octylmethacrylat, n-Dodecylmethacrylat, Tetradecylmethacrylat und Hexadecylmethacrylat sowie die entsprechende Acrylaten und Mischungen davon; und/oder
das oder die Monomere M_{F} ausgewählt sind aus 2-Hydroxyethyl(meth)acrylat, 2-Hydroxypropyl(meth)acrylat, 3-Hydroxypropyl(meth)acrylat, 4-Hydroxybutyl-(meth)acrylat und Mischungen davon; und/oder
das oder die Monomere M_{P} ausgewählt sind aus 2-Isocyanatoethyl(meth)-acrylat, den Additionsprodukten von 1 Mol 2-Hydroxyethyl(meth)acrylat oder 2-Hydroxypropyl(meth)acrylat mit jeweils 1 Mol eines Diisocyanats, Hexamethylen-1,6-diisocyanat (HMDI), 2,2,4-Trimethyhexamethylen-1,6-diisocyanat (TMDI) Isophorondiisocyanat (IPDI), 2-Isocyanatoethylmethacrylat (IEMA) und Mischungen davon; und/oder
das oder die Monomere M_{H} ausgewählt sind aus 2-(Meth)acryloyloxypropyldi-hydrogenphosphat, 2-(Meth)acryloyloxyethyldihydrogenphosphat, 4-(Meth)-acryloyloxybutyldihydrogenphosphat, 6-(Meth)acryloyloxyhexyldihydrogen-phosphat und 10-(Meth)acryloyloxydecyldihydrogenphosphat, Vinylphosphonsäure, 4-Vinylphenylphosphonsäure, 4-Vinylbenzylphosphonsäure, 2-(Meth)-acryloyloxyethylphosphonsäure, 2-[4-(Dihydroxyphosphoryl)-2-oxa-butyl]-acrylsäureethylester und Mischungen davon; und/oder
das oder die Monomere M_{CS} ausgewählt sind aus (Meth)acrylsäure, Itaconsäure, Crotonsäure, Maleinsäure und Fumarsäure sowie deren Halbester, 2-(Hydroxymethyl)acrylsäure, 4-(Meth)acryloyloxyethyltrimellitsäure, 10-(Meth)acryloyloxydecylmalonsäure, 2-(Meth)acryloyloxymethylbernsteinsäure, 4-Vinylbenzoesäure und Mischungen davon.

10. Dentalwerkstoff nach einem der Ansprüche 1 bis 9, bei dem die Base zur Neutralisation des Copolymers A_{P} oder B_{P} oder der Mischung von A_{P} und B aus LiOH, KOH, NaOH, NH₄OH, organischen Aminen, vorzugsweise Ethylamin, n-Butylamin, Dibutylamin, Trimethylamin, Tributylamin (TBA), Triethylamin (TEA), N-Methylmorpholin, oder Dimethylcyclohexylamin, Aminosäurederivaten, vorzugsweise Lysinmethylesterdihydrochlorid, Argininhydrochlorid oder Glycinethylester, OH-funktionalisierten Aminen, vorzugsweise Methyldiethanolamin, Diethylethanolamin, Dimethylethanolamin, Tris(hydroxymethyl)aminomethan, Ethanolamin, Diethanolamin, Triethanolamin (TEOA), Butanolamin, Dibutanolamin, 3-Dimethylamino-2-propanol (DMAP) oder 1,3-Diamino-2-propanol (DAP), höhermolekularen ethergruppenhaltigen Aminen, vorzugsweise Aminogruppen-terminierten oligomeren Ethylenoxid- oder Propylenoxid-Oligomeren, und Mischungen davon ausgewählt ist.

11. Dentalwerkstoff nach einem der Ansprüche 1 bis 10 bei dem die Base oder Basenmischung in einer Menge von 20 bis 90 mol-%, bevorzugt von 25 bis 80 mol-% und besonders bevorzugt von 30 bis 75 mol-% bezogen auf den Gehalt an stark sauren Haftmonomeren M_{H} zugesetzt wird.

12. Dentalwerkstoff nach einem der Ansprüche 1 bis 11, bei dem das Copolymer A_{P} und/oder B_{P} eine zahlenmittlere Molmasse von 5.000 bis 60.000 g/mol, bevorzugt von 10.000 bis 50.000 g/mol und besonders bevorzugt von 15.000 bis 40.000 g/mol aufweist, gemessen mittels Gelpermeationschromatographie.

13. Dentalwerkstoff nach einem der Ansprüche 1 bis 12, der
- 10 bis 50 Gew.-%, bevorzugt 15 bis 45 Gew.-% und besonders bevorzugt 20 bis 35 Gew.-% mindestens eines Copolymers A_{P} und ggf. B_{P,}
- 50 bis 90 Gew.-%, bevorzugt 55 bis 85 Gew.-% und besonders bevorzugt 65 bis 80 Gew.-% Dispersionsmittel und ggf.
- 10 bis 70 Gew.-%, vorzugsweise 15 bis 60 Gew.-% und besonders bevorzugt 20 bis 55 Gew.-% Wasser enthält,
jeweils bezogen auf die Gesamtmasse des Dentalwerkstoffs, wobei der Wassergehalt ggf. auf die Dispersionsmittelmenge angerechnet wird.

14. Dentalwerkstoff nach einem der Ansprüche 1 bis 13 zur intraoralen Verwendung bei der Restauration geschädigter Zähne, vorzugsweise als dentale Adhäsiv oder Schmelz-Dentin-Adhäsiv.

15. Nicht therapeutische Verwendung eines Dentalwerkstoffs nach einem der Ansprüche 1 bis 13 als Adhäsiv bei der Herstellung oder Reparatur von Dentalrestaurationen.
